Europäisches Patentamt

European Patent Office

Office européen des brevets

(11) Publication number: **0 426 924 A1**

## EUROPEAN PATENT APPLICATION

(21) Application number: 89403103.8

(51) Int. Cl.⁵: **A61K 37/02**, A61K 35/28

(22) Date of filing: 09.11.89

(43) Date of publication of application:
15.05.91 Bulletin 91/20

(84) Designated Contracting States:
AT BE CH DE ES FR GB GR IT LI LU NL SE

(71) Applicant: CELLENA (CELL ENGENEERING) A.G.
Lohwisstrasse, 12
CH-8123 Ebmatingen(CH)

(72) Inventor: Pierpaoli Walter
Via Luserte2
6572 Quartino-Magadino(CH)
Inventor: Neri, Paolo
Via Delle Cantine 15
53100 Siena(IT)

(74) Representative: Gutmann, Ernest et al
S.C. Ernest Gutmann - Yves Plasseraud 67,
boulevard Haussmann
F-75008 Paris(FR)

(54) Production of metal transporter compositions for facilitating the engraftment of histo-incompatible bone marrow and controlling the immune reactions in the recipient host.

(57) The invention pertains to a process for the production of a pharmaceutical composition effective for controlling in a recipient host mammal, particularly man, immune reactions of the type that are involved in graft of foreign tissue or cells, particularly bone marrow cells of allogeneic or even xenogeneic origin, or in immunodeficiency-linked diseases, which pharmaceutical composition is characterized by an active principle consisting of a metal, preferably iron, in binded or complexed form with a molecule or group of molecules which allow for the possible active transport in vivo of said metal across or through cell membranes, particularly bone marrow cell membranes. The metal transporting molecules is advantageously selected from transferrin, lactoferrin or conalbumin.

EP 0 426 924 A1

# PRODUCTION OF METAL TRANSPORTER COMPOSITIONS FOR FACILITATING THE ENGRAFTMENT OF HISTO-INCOMPATIBLE BONE MARROW AND CONTROLLING THE IMMUNE REACTIONS IN THE RECIPIENT HOST.

The invention relates to the production of compositions, particularly pharmaceutical compositions, containing an active principle capable of controlling the immune reactions of a host against allogenic cells or tissues or of immunocompetent cells against an immunoincompetent or immunosuppressed host, particularly those immune reactions which are involved in the so-called host versus-graft reaction (HVGR) and so-called graft versus-host reaction (GVHR), as well as immune reactions which are brought into play when the host is transplanted with allogeneic or xenogeneic incompatible bone marrow.

Testing procedures are available for demonstrating the capability of active principles, e.g. bone marrow factors, of controlling immunological reactions (HVGR and GVHR) in a lethally irradiated recipient injected with immunogenetically different bone marrow from an allogeneic or even xenogeneic donor : see for instance the article of W. Pierpaoli et al, titled "Experimental manipulations and marrow-derived factors which affect the outcome of bone marrow transplantation across the H-2 barrier in lethally irradiated mice" (J. Clin. Lab. Immunol. (1985), 16, 115-124). More particularly, it has already been found that such bone marrow cell factors were liable of facilitating engraftment of foreign cells or tissue (allogenic or xenogeneic) in a host, when they were administered to said host according to a determined protocol or regimen including a total body irradiation procedure designed to destroy the host's own immune system, thus to provide for an easier substitution in said host of an immune system of foreign origin for its own one.

Bone marrow transplantation (BMT) is a key biological problem. Mastering the conditions of transplantation in a host, while at the same time controlling the host immune responses, particularly the host's HVGR and GVHR, would represent a major advance in therapies involving not only cell- or organ grafts (e.g. liver, kidney, heart, skin and others) but also prevention, alleviation, relief or even cure e.g. of solid tumors, leukemia, of the effects of a number of genetic diseases, age-related diseases and immunodeficiency-linked diseases (like AIDS). The beneficial effects and prospects of allogeneic and even xenogeneic bone marrow transplantation for prevention and curing of such diseases is well documented see for instance following publications whose content shall be deemed to form part of the background disclosed in the instant application :

"Bilan actuel de la greffe de moelle osseuse allogénique" (Present overview of the allogenic bone marrow graft) of E. Gluckman, Path. Biol., 1980, 28, n° 1, 5-7 ;

"Future of bone marrow transplantation in oncology" of Alexander Fefer, Int. J. Radiation Oncology Biol. Phys. May 1982, vol. 8, pp. 949-950 ;

- "A review of the current status and techniques of allogeneic bone marrow transplantation for the treatment of leukemia" of H.G. Prentice, J. Clin. Patol. 1983 ; 36 : 1207-1214 ;

- "Allogenic Bone Marrow Transplantation : Current Status and Future Directions" by Richard J. O'Reilly, Blood, vol. 62. No. 5 (November 1983), pp.941-964 ;

"Role of Bone Marrow Transplantation in the treatment of Hematologic Malignancies and Solid Tumors : Critical review of Syngeneic, Autologous, and Allogeneic Transplants" by R.E. Champlin et al, Cancer Treatment Reports, vol. 68, n° 1, January 1984 ;

- "Clinical Bone Marrow Transplantation Current Status" by K. Atkinson, Transplantation Proceedings, 1984, vol. XVI, No. 4 ;

- "Current Status of Bone Marrow Transplantation" by E.D. Thomas, Transplantation Proceedings, vol. XVII, No. 1 (February) 1985 ;

- "Bone Marrow Transplantation" by R.P. Gale et al (198..) CRC Critical Reviews in Oncology/Hematology, vol. 2, Issue 3 ;

The present invention stems from the discovery that the control of immune reactions of a recipient host in relation to allogeneic or xenogeneic cells or tissue of a donor host can be achieved by engrafting in or administering to said recipient host bone marrow (transplanted bone marrow cell) of said donor host and by causing in the recipient host a sufficient level of iron or another metal capable of acting in a similar fashion to be maintained, in order to allow for the proliferation of the transplanted bone marrow cell in said recipient host, said iron or other metal being supplied in a form allowing for their active transport in vivo through bone marrow cell membranes.

It has been found that the presence in the recipient host of said iron or metal under said form can also produce a powerful effect on the engraftment of allogeneic, as well as of xenogeneic bone marrow in said host, more generally control immune responses in the host against such xenogeneic or allogeneic cells or tissue.

By way of a first example as to the form of the iron or metal to be used reference is made to the complexes which they can form with glycoproteins or proteins occuring naturally in animals (or animal products, e.g. milk or eggs) and which are capable or reversibly binding said iron or metal and of transporting them across cell membranes and/or preventing loss of that iron or metal from the host's body.

The invention thus relates more particularly to the production of a pharmaceutical composition effective for controlling in a mammal, particularly man, immune reactions of the type that are involved in graft of foreign tissue or cell, particularly bone marrow cells of allogeneic or even xenogeneic origin, or in immunodeficiency-linked diseases, which pharmaceutical composition is characterized by an active principle consisting of a metal, preferably iron, in binded or complexed form with a molecule or groups of molecules which allow for the possible active transport in vivo of said metal across or through cell membranes, particularly bone marrow cell membranes.

It goes without saying that the expression "second host" as used hereabove includes both the host from which said allogeneic or xenogeneic cells or tissue originated and any other host being syngeneic or histocompatible with the preceding one.

A first advantageous class of such metal binding molecules or groups of molecules consist of glycoproteins or proteins selected among those which have in vivo specificity for said metal and to which correspond cell- or tissue- receptors, particularly membrane receptors which allow binding of the glycoprotein-metal complex on said cell or tissue and the carrying thereof into said cell or tissue with an attendant release of the metal therein.

Major representatives of that class of glycoproteins or proteins (metal-transport proteins or glycoproteins) consist of transferrins.

Transferrins are a class of two-sited, single chain, metal-binding proteins, widely distributed in physiological fluids and cells of vertebrates.

Each of the transferring consists of a single polypeptide chain, of molecular weight in the range 76.000-81.000, which contains two similar but not identical binding sites.

According to the tissue origin, serum, lacto- and ovotransferrin can be distinguished, although all these proteins share common molecular features.

If the shape of human serum transferrin is approximated by an ellipsoid of revolution, then the ratio of major radii to minor is 2:1 for iron-saturated transferrin, and increases to 2.5:1 or 3:1 when the protein is freed from iron.

The isoelectric point of serum transferrin is on the acid side of neutrality.

The transferrins are all glycoproteins.

Human serum transferrin contains about 5 % carbohydrate, linked to the protein in two identical and nearly symmetrical branched heterosaccharide chains. It has a molecular weight of about 80,000. 1 mg of the iron-saturated protein contains about 1.4 $\mu$g iron.

The complete amino acid sequence of human plasma transferrin has recently been established by at least three groups using CNBr cleavage (CNBr) and by complementary DNA (cDNA) methods (MacGillivray, R.T.A., et al. "The complete amino acid sequence of human serum transferrin". Proc. Natl. Acad. Sci. USA 79 : 2504-2508, 1982 and Uzan, G. et al. "Molecular cloning and sequence analysis of cDNA for human transferrin. Biochem. Biophys. res. Commun. 119 : 273-281, 1984 and Yang, F. et al. "Human transferrin : cDNA charaterization and chromosomal localization". Proc. Natl. Acad. Sci. USA 81:2752-2756, 1984). It is composed of 678 aminoacid residues, which together with the two-N-linked oligosaccharide chains exhibit a calculated molecular weight of 79,570 (of which 6 % is contributed by the glucosidic moiety: MacGillivray, R.T.A. et al and Uzan G. et al, "Molecular cloning and equence analysis of cDNA for human transferrin". Biochem. Biophys. Res. Commun. 119 : 273-281, 1984). Wiliams J. ("The evolution of transferrin", Trends Biochem. Sci, 7 : 394-397, 1982) has suggsted the importance of sulfhydryl groups in stabilizing the iron-binding site and has traced their evolutionary development to the 17 disulfides found in human transferrin.

For a general review of the status of general knowledge about transferrins see the general publication titled "The Physiology of Transferrin and Transferrin Receptors" by Helmut A. Huebbers and Clement A. Finch in Physiological Reviews, vol. 67, n° 2, April 1987.

Procedures for obtaining transferrin, particularly transferrin of human origin in a biologically pure state have been disclosed in that publication. Preferred purification procedures are either based on physico-chemical based separation steps followed by selective fixation on matrix-bound antibody or matrix-bound receptor.

Purified iron-saturated transferrin in a substantially biologically pure state is substantially free of serum albumin proteins.

Though preference ought to be given to glycoproteins or proteins which accomodate and bind iron and which are able to deliver said metal into the relevant cell or tissue, one may also contemplate for use in the production of the above-mentioned pharmaceutical compositions, of any of such glycoproteins or proteins which are able to operate as in vivo transport agents of other metals, e.g. gallium, copper, chromium, cobalt, manganese, vanadium, aluminium, terbium, indium or platinum.

Preferably said metal-binding glycoprotein or protein is of the same animal species as the host to be engrafted, e.g. consists of a transferrin of human origin when the pharmaceutical composition is for use in man.

Other preferred glycoproteins which can be resorted to consist of, e.g. lactoferrins or conalbumin, in substantially biologically pure state too, also loaded with metal, e.g. iron.

As concerns structural and physicochemical characteristics of such other glycoproteins reference is made, by way of example for lactoferrins, to overview articles by Anderson, B.F., Baker, H.M., Dodson, E.J., Norris, G.E., Rumball, S.V., Waters, J.M. and Baker, E.N. (1987) Proc. Natl. Acad. Sci. USA 84, 1769-1773 Gorinsky, B., Horsburgh, C., Lindley, P.F., Moss, D.S., Parkar, M. and Watson, J.L. (1979) Nature 281, 157-158.

Reference is also more particularly made to those metal-binding glycoproteins which provide positive responses in the following testing procedures which aim at assessing both qualitatively and quantitatively the biological properties sought in mammals, e.g. mice.

In the preceding text reference has essentially be made to metal-transport proteins or glycoproteins. Also fully synthetic iron-carrier molecules with non-proteic character may be active for in vitro or in vivo iron transport and are thus likely to be used in a system to achieve engraftment of allogeneic or xenogeneic bone marrow in the relevant host. Reference is made for instance to salicylilaldehyde isonicotinoyl hydrazone which is capable of supplying iron to cells in vitro, possibly without using physiological pathways characteristic of the natural metal-transport proteins or glycoproteins.

A method for causing stable engraftment or transplantation in a recipient host of bone marrow cells of a donor host comprises administering to said recipient on the one hand, the bone marrow to be transplanted from said donor host and, on the other hand, and in appropriate time relationship; doses of iron or said other metal in the above said form, effective to cause said iron or other metal to be maintained at a level sufficient to allow for the proliferation of the transplanted bone marrow in the recipient host. Most preferably the endogeneous immune defenses of said receiver host are destroyed at least in part prior to the above mentioned administrations of bone marrow and iron or metal transporting molecules, in order to improve the subsequent engraftment.

The above method is generally applicable to the further engraftment in the recipient host of allogeneic or xenogeneic cells other than bone marrow, or of allogeneic or xenogeneic tissue or organs (e.g. liver, heart, kidney) of a donor host, whereby the administration as set forth above of the bone marrow to be transplanted, preferably of the same or syngeneic donor host, and of the iron transporters must be appropriately adjusted relative to the time of engraftment of said other cells, tissue or organs.

The invention then further relates, particularly in relation to the above method alternative, to the composition of matter comprising bone marrow of a donor host on the one hand, and of the metal transporting molecules on the other, particularly for use in the above methods.

Further aspects of the invention will appears as the description of the "biological activities" proceeds as set forth hereinafter.

## BIOLOGICAL ACTIVITIES

1) Materials and methods generally applicable to the testing of the capability of an active principle to control the immune reactions of a host under circumstances referred to above.

Animals :

The animals used are mice, which have been bred under specific pathogen-free conditions and then maintained, as adults, under strictly standardized and controlled hygienic conditions. However, no special precautions need be taken to avoid pathogenic bacteria or viruses.

Donors or recipients of bone marrow can be inbred young adult (8-16 weeks old) C57BL/6, BALB/C and C3H/He mice. Other mice can be used too : see examples.

Rats can be used too : e.g. Lewis or Long Evans inbred strains. They can be used as donors of xenogeneic marrow to mice.

4

Preparation of bone marrow cell suspensions (of donors) :

Mice (donors) are killed by cervical dislocation ; the long bones (humeri, tibiae and femurs) are isolated and cut at the extremities. Ice-cold TC 199 medium is flushed repeatedly through the bone cavities by a syringe with a needle fitting the bone size. The pooled marrow is gently dispersed by a needleless syringe and filtered through gauze. The cells are then sedimented by low-speed centrifugation, the supernatants discarded and the cells are resuspended in TC 199 medium. The final cell suspension is adjusted to the desired number and volume. Quantities varying from $10 \times 10^6$ to a maximum of $40 \times 10^6$ cells per mouse are injected intravenously. Trypan-blue exclusive tests show that over 95 % of the cells are normally viable just before their inoculation.

Irradiation of recipients :

A dose of 850 to 1000 rads total body irradiation (TBI) is given to the recipients, depending on the known strain sensitivity to irradiation. This dose causes death of all untreated mice within 8-15 days. The irradiation apparatus is for instance a Cobalt Gammatron (6000 Curie). Field size is $30 \times 30$ cm, main focus distance is 90 cm. No filters are used.

Transplantation of allogeneic or xenogeneic bone marrow and treatment with the active principle : :

The active principles are prepared in sterile isotonic saline (water solution of 8.5 g NaCl per liter) and, where appropriate, stored frozen in aliquots suitable for mice : aliquots comprise e.g. 50 or 500 $\mu$g in 0.2 ml of the active substance to be tested (0.2 ml per mouse per day).

The irradiated mice to be transplanted (bone marrow transfer circa 24 hours after TBI) are injected intravenously with the wished number (10 to $20 \times 10^6$) of donor bone marrow cells suspended in volumes of 0.05 ml. From the day of bone marrow transfer and thereafter, the selected dosage of active principle under study is administered daily over 9-10 days, intra-peritoneally.

Tests for chimerism :

a) Hemoglobin migration pattern

At monthly intervals after TBI, when appropriate (when donor and recipient mice display clear differences in the molecular structure and composition of their hemoglobin, e.g. C57Bl/6 (homogeneous) and BALB/c or C3H/He (heterogeneous) hemoglobin) all mice are individually tested for chimerism of the erythroid cell line. A few drops of blood are taken from the retroorbital plexus, suspended in heparinized saline and washed repeatedly. The washed blood cells are hemolyzed in 1:5 cell water volume. The pattern of hemoglobin migration is examined by cellulose-acetate electrophoresis. The strips are stained with Amido Black. Engraftment of the donor erythropoietic line corresponding to complete chimerism is tied to the following observations in said test : for example, when the chimeric mouse is a C57Bl/6 transplanted with bone marrow from BALB/c (BALB/c → C57Bl/6), the erythropoietic line in the chimera is that belonging to BALB/c. Hemoglobin migrates as double-band. Conversely, hemoglobin migrates as a single-band when, for example, the hemopoietic chimeras are C3H/He or BALB/c mice transplanted with bone marrow from C57Bl/6 mice (see also W. Pierpaoli and G. Maestroni : The facilitation of enduring engraftment of allogeneic bone marrow and avoidance of secondary disease in mice. Cellular Immunology 52, 62-72, 1980).

b) Skin grafts

After cross-transplantation of bone marrow from donor mice to recipient allogeneic mice, donor skin is grafted on groups of allogeneic marrow recipients. Grafting is by conventional technique, e.g. as published in W. Pierpaoli, G. Maestroni and E. Sache : Enduring Allogeneic Marrow Engraftment via Nonspecific Bone-Marrow-Derived Regulating Factors (MRF). Cellular Immunology 57, 219-228 (1981) ; the corsets are removed after 8-10 days and the viability of the grafts is checked daily. The graft is considered as rejected when the first signs of infiltration, oedema and induration appear. In contrast the graft is considered as accepted only when none of these signs is discernible and luxuriant hair grows on the transplanted skin.

c) H-2 typing

Additional tests to prove the persistent presence of donor-type hemopoietic line in our chimeras were those described in : G. Maestroni, W. Pierpaoli and R. Zinkernagel, Immunoreactivity of long lived H-2 incompatible irradiation chimeras (H-2$^d$) H-2$^b$). Immunology 46, 252-260, 1892. Mixed lymphocite reactions and anti-H-2 typing were performed in the mouse chimeras as described in the paper above.

The invention is further illustrated by the following test assays carried out with three iron-loaded representative glycoproteins. The method involved transplantation of allogeneic (incompatible across the major histocompatibility barrier) bone marrow in mice and the achievement of chimerism by using iron-loaded transferrin (siderophillin), lactoferrin and conalbumin both of which carried iron too. Transferrin was a commercial production extracted from plasma. Lactoferrin and conalbumin were extracted products obtained from human milk and egg albumin.

It is to be understood that similar experiments with rats, rabbit or Guinea-pig can be carried out to produce xenogeneic (inter-species) chimerism.

Mice used :
C57BL/6J (black) inbred females, histogenetically known as H-2$^b$.
BALB/cJ (white albino) inbred females known as H-2$^d$.
C3H/HeJ (light brown) inbred females known as H-2$^k$.
Active Principle used :
- Transferrin, bovine, iron-free (approx. 98 %) SIGMA No. T-5761, lot. No. 118F-9342 ;
- Transferrin, human, Holo-form (siderophilin) (approx. 98 %), SIGMA No. T-4778 lot. No. 67F9458 ;
- Conalbumin, iron-complex, Type II : from chicken egg white, SIGMA C-0880, lot. No. 104F8065 ;
- Lactoferrin, iron-saturated, from human milk, approximatively 90 % (SIGMA No. L-3770, Lot no: 98F3924).
Irradiation :
Total body irradiation (TBI) with 959 rad (supralethal).

Bone marrow was isolated from the long bones (femurs, tibiae) of the mice killed by cervical dislocation. The cells were dissociated and centrifuged to remove the medium in which they had been isolated and suspended (bone marrow supernatant BM-SN). They were then counted and adjusted to the desired concentration and injected intravenously (15 millions per mouse injected) via the retroorbital venous plexus, under acute ether anaesthesia in 0.5 ml volume (medium TC 199 with antibiotics).

The bone marrow was injected about 24 hours after TBI, to give time to the irradiated bone marrow of the recipient to vanish out or decrease.

Substances :

Siderophillin (Iron-loaded transferrin), lactoferrin or conalbumin were prepared in sterile saline and stored frozen in aliquots suitable for groups of 10 mice each. From the day of bone marrow transfer (24 hours after TBI), and after the bone marrow, they were injected intraperitoneally every day, in the afternoon, for 10 days, at 100 μgrams/mouse/day.

The mice were kept indefinitely without any further protection until they either became chimeras or died. They were considered as being chimeras when it was found that they carried the bone marrow from the donor. The hemoglobin migration assay with gel electrophoresis of single mouse hemoglobin was used too. It involved checking whether the erythroid line was of donor or recipient character or by skin grafting. In the latter cases irradiated and BM-transplanted mice are skin-grafted with skin from the bone marrow donor. When the skin was retained and accepted (the fur was growing, for example clearly white (BALB/c) on black (C57BL/6) it meant that the mice were chimeras. They then also carried successfully the bone marrow from the donor. This was the most important and clear-cut proof for complete hemopoietic chimerism without immunodeficiency became chimerism was allospecific and the chimeras were perfectly able to reject allografts (skin) from an unrelated donor (e.g. C3H/He, H-2$^k$ mice) within a normal time (10-14 days).

There follows the results (in the following table) of assays carried out with :

The table clearly witnesses the following facts:

- the iron-loaded glycoproteins used protect the mice against both the previous lethal irradiation which they had undergone and the transplantation of allogeneic material
- the surviving mice accepted that allogenic material as if it had been of its own: they became full chimeras as defined above.

TABLE .

| Treatment with iron-loaded transferrin (siderofillin), lactoferrin and conalbumin in supralethally irradiated mice transplanted with bone marrow from genetically histoincompatible donors (BALB/cJ→C57BL/6J; H-$2^d$→H-$2^b$) produces engraftment of allogeneic bone marrow and permanent allospecific graft-versus-host free hemopoietic chimerism | | | | | |
|---|---|---|---|---|---|
| Groups | Treatment | Dose/day | No of mice | Survival at 4 months | Chimerism |
| A | Human serum albumin | 100 μg | 29 | 0 | - |
| B | Iron-free Transferrin | 100 μg | 20 | 0 | - |
| C | Iron-loaded Transferrin | 100 μg | 20 | 20 (100%) | 100% |
| D | Iron-loaded Lactoferrin | 50 μg | 10 | 9 (90%) | 100% |
| E | Iron-loaded Conalbumin | 100 μg | 10 | 7 (70%) | 100% |

The compositions of the invention are suitable for use in the many areas which have already been referred to earlier. Be it recalled by way of reminder that these compositions are suitable particularly for the treatment of the following classes of diseases, all of which would benefit of bone marrow in vivo transplantation :
Aplastic anemia ; agranulocystosis ;
Thalassemia ;
Immunodeficiency diseases (AIDS, agammaglobulinemia, etc.) ;
Leukemias (Myeloblastic, lymphoblastic, erythroblastic, etc.)
Myelomas ;
Solid tumors, carcinomas, adenocarcinomas ;
Genetic diseases ;
Organ transplantation after BMT from man or animals (pig, monkey, etc.) (Acceptance of liver, heart, kidneys without rejection reaction).

See also general indications supplied by Gluckman E. in its article titled "Bilan actuel de la greffe de moelle osseuse allogénique" (General overview on the graft of allogenic none marrow) published in Path. Biol. 1980, 28, No. 1, 5-7. These indications are also applicable here.

The invention finds use preferably in the graft of allogeneic (histoincompatible non-HLA-matched) BMT, whereby the difficulties linked to the finding of a donor should be circumvented to a great extent. The invention is not limited to compositions for use in the graft of allogeneic BMT only. Their use is to be contemplated in any system aiming at facilitating the engraftment of any type of bone marrow in any type of mammal including man. The engraftment of xenogeneic (inter-species, e.g. pig or primate to man) BMT is believed to be within reach of the present invention.

Though the way of administering the composition of the invention and its coupling with the steps involving abrogating the endogeneous immunitary system in the host to be transplanted with allogeneic on xenogeneic bone marrow should rest with the clinicians, it nevertheless remains that the abovesaid abrogation should normally be caused to take place prior to the transplantation.

Noteworthy is the fact that irradiation is not the only system for abrogating immunity in the receiving host prior to transplanting the bone marrow. The systems for immunosuppression in the host may also bring into play cytostatic drugs like cyclophosphamide alone or combined with irradiation, or also total lymphoid irradiation (TLI), irradiation only of the lymphatic organs in order to prevent large irradiation damage (lungs, intestine, etc.). Any cytostatic and immunosuppressive drug, e.g. cyclosporin may be given alone or in combination with irradiation to condition the recipient to the transfer of bone marrow.

Neither are the uses of the compositions of the invention limited to the transplantation of bone marrow only. They become applicable whenever a transfer into the treated host of a new immunological system is required, e.g. for inducing rejection by the host of leukemia cells, solid tumors. Another important use of the invention is in xenogeneic (inter-species) BMT, for example when the donor of bone marrow and organs (liver, heart, kidneys) is piglet or a primate (monkeys) and the receiver is man.

Alternatives in the moments at which the iron-loaded carrier glycoproteins or proteins are to be administered are contemplated too. They may also be administered to the donor, prior to the transfer of its bone marrow to the recipient. The administration of said iron loaded proteins or glycoproteins to the donor is likely to favor the engraftment-capacity of the bone marrow in the recipient due e.g. tolerance induced towards the donors of none marrow and organs.

Iron-loaded glycoproteins like siderophilin may also be added to bone marrow cultures, to pre-incubate in vitro the donor bone marrow for variableperiods (hours or days) before its inoculation in the recipient. This procedure may change and/or improve the engraftment capacity of the donor bone marrow and enhance induction of GvHd-free chimerism. This pre-induction may be or not associated to the post-transplantation treatment with iron-carrier proteins.

The compositions of the invention may be administered by any route normally used to enhance the host non- responsiveness to the foreign (allogeneic or xenogeneic bone marrow and/or organs). Though oral or rectal routes may be contemplated, the preferred ones remain the parenteral routes (intravenous or intramuscular injections).

Though this should not be construed in any limitative manner whatsoever, daily doses of iron-loaded glycoprotein or protein administered to the host after the engraftment of bone marrow and/or organs sought to be grafted has been achieved, should normally range from about $5 \times 10^{-6}$ to about $500 \times 10^{-6}$ mM per kg body weight of the host. Treatments of that type should normally last from 10 to 30 days after said engraftment.

However, the treatment with the iron-loaded protins may also start days, weeks or months after transplantation of bone marrow, in all those cases in which the transplanted individualshows signs or symptoms of an ill-functioning hemopoietic system (anemia, leucopemia, trombocytopemia or of graft versus host disease and immunological deficiencies (parasitic, viral or bacterial infections).

It should finally be emphasized that the invention is not limited to the use, for the production of the abovesaid pharmaceutical compositions, of the natural full iron-loaded glycoproteins of natural origin, essentially as contemplated hereabove.

The active principles of these compositions may also consist of corresponding genetically engineered proteins, e.g. of the expression products in appropriate host cells of the cDNAs (or of fragments of said cDNAs) obtained from RNAs themselves obtained from the natural live source material of said glycoproteins. Needless to say that the genetically engineered proteins should then also retain the capabilities of the natural glycoproteins to bind iron or other relevant matal and to exhibit the biological activities thereof, e.g. capability of binding to the corresponding natural receptors and/or to produce a positive response in the biological assays referred to above.


Example


Tentative protocol for transplantation of allogeneic (HLA or non-HLA-compatible) or xenogeneic bone marrow in man.

For example, a person with leukemia or a solid tumor, e.g. lungs, stomach).

- Preparation of bone marrow cell suspension from a donor (man, piglet, monkey) with or without donor-recipient HLA-typing. No manipulation is performed on the donor bone marrow before inoculation to remove cellular components (e.g. T cells). The procedure is similar to that described in the literature for preparation of donor bone marrow.

- Lethal irradiation of the recipient (total body or total lymphoid irradiation) alone or combined with cystostatic, immunosuppressive drugs.

- 24 to 48 hours later, intravenous injection of the donor bone marrow.

- From day of marrow transplantation, treatment with 5 to 500 mM/kg human siderophilin, lactoferin or alternatively conalbumin for 10 to 30 days.

- Clinical and hemobiological follow-up of the patient to assess :

a) donor bone marrow engraftment ;

b) absence of secondary, acute or chronic GvHD ;

c) evaluation of hemopoietic chimerism by some of the many tests available (donor-type erythrocytes, macrophages, leucocytes, lymphocytes, HLA-typing, etc.) ;

d) clinical evolution of leukemia or of the solid tumor in the transplanted patient ;

e) in case of symptoms of GvHD, further treatment for days or weeks with iron-carrier proteins.

In case an organ (liver, heart, kidney) must be transplanted, the patient must be hemopoietic chimera of the bone marrow from the same donor of the organ before or when the organ is transplanted. As a matter of example, bone marrow and the liver are removed from a person dying after an accident (alternativelt, inbred piglets can be used as donor or xenogeneic bone marrow or organs). The patient (e.g. with acute heart or liver failure) is treated with TBI, TLI or immunosuppressive drugs, infused with donor bone marrow and transplanted with liver or heart from the same boine marrow donor. the bone marrow and organ-transplanted patient is then treated with iron-carrier proteins as above. One can reasonably expect that if treatment with iron-carrier-proteins results in achievement of hemopoietic chimerism free of Graft versus Host Disease (GvHD), the patient should accept the transplanted organ without any sign of immune rejection against the organ. It is possible that a partial (mixed) chimerism is sufficient to achieve transplantation tolerance, in which the organ-transplanted patient maintains a part of his own hemopoietic tissue in symbiosis with the accepted allogeneic bone marrow (mixed chimerisms) from the donor of the organ. In this case it may be possible to drastically reduce the immunuppressive regimen before bone marrow transplantation and treatment with iron-carrier-proteins.


## Claims

1. Process for the production of a pharmaceutical composition effective for controlling in a recipient host mammal, particularly man, immune reactions of the type that are involved in graft of foreign tissue or cells, particularly bone marrow cells of allogeneic or even xenogeneic origin, or in immunodeficiency-linked diseases, which pharmaceutical composition is characterized by an active principle consisting of a metal, preferably iron, in binded or complexed form with a molecule or groups of molecules which allow for the possible active transport in vivo of said metal across or through cell membranes, particularly bone marrow cell membranes.

2. The process of claim 1 wherein said molecule consists of at least one metal-binding glycoprotein or protein, loaded with metal, preferably iron, in binded or complexed form therein, said metal binding glycoprotein or protein being selected among those which have in vivo specificity for said metal and to which correspond cell-or tissue- receptors, particularly membrane receptors, particularly membrane receptors which allow binding of the glycoprotein-metal complex on said cell or tissue and the carrying thereof into said cell or tissue with an attendant release of the metal therein.

3. The process of claim 2, wherein said glycoprotein is a transferrin, preferably human transferrin.

4. The process of claim 2, wherein said glycoprotein is a lactoferrin.

5. The process of claim 2, wherein said glycoprotein is a conalbumin.

6. The process pf any of claims 1 to 5, wherein said metal is iron.

7. A composition of matter comprising bone marrow of a donor host on the one hand, and of the metal transporting molecules, on the other hand, said metal, preferably iron being in binded or complexed form with a molecule or group of molecules which allow for the possible active transport in vivo of said metal across or through cell membranes, particularly bone marrow cell membranes.

8. A composition of matter of claim 7 wherein said molecule consists of at least one one metal-binding glycoprotein or protein, loaded with metal, preferably iron, in binded or complexed form therein, said metal binding glycoprotein or protein being selected among those which have in vivo specificity for said metal and to which correspond cell- or tissue- receptors particularly membrane receptors which allow binding of the glycoprotein-metal complex on said cell or tissue and the carrying thereof into said cell or tissue with an attendant release of the metal therein.

9. The composition of matter of claim 8, wherein said glycoprotein is a transferrin, preferably human transferrin.

10. The composition of matter of claim 8, wherein said glycoprotein is a lactoferrin or a conalbumin.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | BIOCHIMICA ET BIOPHYSICA ACTA, vol. 583, 1979, pages 483-490, Elsevier/North-Holland Biomedical Press; J.W. LARRICK et al.: "Transferrin receptors on human B and T lymphoblastoid cell lines" * Materials & Methods * | 1-3 | A 61 K 37/02<br>A 61 K 35/28 |
| X | CHEMICAL ABSTRACTS, vol. 93, no. 5, 4th August 1980, page 352, abstract no. 39889s, Columbus, Ohio, US; J. MAZURIER et al.: "Comparative study of the iron-binding properties of human transferrins. I. Complete and sequential iron saturation and desaturation of the lactotransferrin", & BIOCHIM. BIOPHYS. ACTA 1980, 629(2), 399-408 * Whole abstract * | 1-3 | |
| A | FEDERATION PROCEEDINGS, vol. 41, no. 4, 1982, abstract no. 3894; C.P. GARRISON et al.: "Serum transferrin as a prognostic indicator in bone marrow tranplant patients" | | TECHNICAL FIELDS SEARCHED (Int. Cl.5)<br><br>A 61 K |

The present search report has been drawn up for all claims

| Place of search | Date of completion of the search | Examiner |
|---|---|---|
| THE HAGUE | 06-07-1990 | ALVAREZ Y ALVAREZ C. |